(19) Europäisches Patentamt / European Patent Office / Office européen des brevets

(11) **EP 4 029 448 B1**

(12) ## EUROPEAN PATENT SPECIFICATION

(45) Date of publication and mention
of the grant of the patent:
**28.01.2026 Bulletin 2026/05**

(21) Application number: **21218059.0**

(22) Date of filing: **29.12.2021**

(51) International Patent Classification (IPC):
**A61B 5/287** (2021.01)     **A61B 5/361** (2021.01)
**A61B 5/367** (2021.01)     A61B 5/06 (2006.01)

(52) Cooperative Patent Classification (CPC):
**A61B 5/367; A61B 5/287; A61B 5/361;
A61B 5/6859; A61B 5/6869; A61B 5/7239;
A61B 5/7264;** A61B 5/0036; A61B 5/062;
A61B 5/066

(54) **LOCAL ACTIVATION DRIVER CLASSIFICATION MAPPING IN ATRIAL FIBRILLATION**

KARTIERUNG DER KLASSIFIZIERUNG VON LOKALEN AKTIVIERUNGSTREIBERN BEI VORHOFFLIMMERN

MAPPAGE DE CLASSIFICATION DE CONDUCTEURS D'ACTIVATION LOCALE EN FIBRILLATION AURICULAIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **04.01.2021 US 202163133723 P
30.11.2021 US 202117537929**

(43) Date of publication of application:
**20.07.2022 Bulletin 2022/29**

(73) Proprietor: **Biosense Webster (Israel) Ltd.
Yokneam 2066717 (IL)**

(72) Inventors:
• **SHA,Qun
Yokneam 2066717 (IL)**
• **NAVRAZHNYKH, Luizetta Vadimovna
Yokneam 2066717 (IL)**

(74) Representative: **Carpmaels & Ransford LLP
One Southampton Row
London WC1B 5HA (GB)**

(56) References cited:
**US-A1- 2014 088 943**

• **TAO SUSUMU ET AL: "Ablation as targeted perturbation to rewire communication network of persistent atrial fibrillation",** vol. 12, no. 7, 5 July 2017 (2017-07-05), pages e0179459, XP055923320, Retrieved from the Internet <URL:https://pdfs.semanticscholar.org/eacd/8abbdd123cd204ea0aba9a062b05c86ae0ca.pdf?_g a=2.250325957.2127306398.1652988071-2043981 183.1624396481> DOI: 10.1371/journal.pone.0179459

• **TAO SUSUMU ET AL: "FUNCTIONAL NETWORKS CREATED BY NONSIMULTANEOUSLY RECORDED ELECTROGRAMS PREDICT ABLATION OUTCOMES OF PERSISTENT ATRIAL FIBRILLATION", HEART RHYTHM,** vol. 16, no. 5, 8 May 2019 (2019-05-08), XP085685119, ISSN: 1547-5271, DOI: 10.1016/J.HRTHM.2019.04.013

Description

## FIELD OF THE INVENTION

This invention relates generally to cardiology, and specifically to cardiac arrythmia.

## BACKGROUND OF THE INVENTION

Atrial fibrillation (AF) is the most common arrythmia, projected to affect 6-12 million people in the United States by 2050 and 17.9 million people in Europe by 2060. Radiofrequency (RF) or irreversible electroporation (IRE) or pulsed field (PF) ablation is a treatment option for AF which acts to change the path of an electric wave in the heart. However, in order to apply the ablation effectively, it is important to locate the source or driver of the AF, and methods for implementing this are known.

AF analysis is for instance described in Tao S, et al.: "Ablation as targeted perturbation to rewire communication network of persistent atrial fibrillation", PLoS ONE, vol. 12, no. 7, 2017-07-05, e0179459.

## SUMMARY OF THE INVENTION

An embodiment of the present invention provides a computer program according to claim 1.

The signals may be unipolar or bipolar voltage or action potential voltage vs. time signals.

Typically, calculating from the signals includes estimating local activation times (LATs) from the signals.

In a disclosed embodiment the heart tissue is part of an atrium, and classifying the atrial fibrillation includes estimating a percentage of remodeling of the atrium.

In a further disclosed embodiment the computer program includes instructions to present to a user a classification of the atrial fibrillation.

The plurality of electrodes may be located on a catheter having a multiplicity of spines.

Typically, the nearest-neighbor electrodes in the plurality of electrodes are separated by less than 3mm.

In another embodiment, averaging respective local efficiency metrics of the nodes consists of generating subgraphs of nodes connected directly to a given node, calculating local efficiency metrics for each of the subgraphs, and averaging the calculated local efficiency metrics.

In yet another embodiment, the computer program includes instructions for configuring the processor to reiterate the steps of generating the graph, and average the respective local efficiency metrics while incrementing the selected mutual information metric threshold, so as to produce a set of ordered pairs of resultant local efficiency and mutual information threshold.

The set of ordered pairs may be analyzed to classify the atrial fibrillation. Typically, analyzing the set of ordered pairs consists of fitting a polynomial to the set, and classifying the atrial fibrillation in response to a first derivative of the polynomial.

In a yet further embodiment, calculating from the signals includes calculating from the signals respective mutual information metrics between all pairs of the electrodes.

There is also provided, according to an embodiment of the invention, an apparatus according to claim 3.

## BRIEF DESCRIPTION OF THE DRAWINGS

The present disclosure will be more fully understood from the following detailed description of the embodiments thereof, taken together with the drawings, in which:

Fig. 1 is a schematic, pictorial illustration of an atrial fibrillation classification system;
Fig. 2 is a flowchart of steps of an algorithm performed by a processor of the system;
Fig. 3 illustrates two histograms;
Fig. 4 is an exemplary schematic regional information graph;
Figs. 5A and 5B are schematic diagrams illustrating subgraphs;
Figs. 6A and 6B are schematic graphs of resultant local efficiency vs. mutual information thresholds;
Figs. 7A, 7B, and 7C are further schematic graphs of resultant local efficiency vs. mutual information thresholds; and
Fig. 8 shows schematic graphs of local efficiency first derivatives vs. mutual information thresholds.

## DETAILED DESCRIPTION OF EMBODIMENTS

Overview

While, for atrial fibrillation (AF), it is important to locate the driver of the AF, it is also important to classify the atrial fibrillation to identify an optimal strategy for RF (radiofrequency) or IRE (irreversible electroporation) /pulsed field (PF) ablation. Areas with significant remodeling, i.e., electrophysiological and/or structural changes in the atrium, may be important in atrial fibrillation maintenance mechanisms. Consequently, knowing whether or not an atrium has been remodeled leads to improvement in the mapping and ablation strategy.

The present disclosure develops a novel strategy for estimating how much remodeling is present in a fibrillating atrium.

A probe, comprising a plurality of electrodes, is inserted into a human patient so that the electrodes contact heart tissue that is undergoing atrial fibrillation. A processor acquires signals from the electrodes, and calculates a mutual information metric between each pair of electrodes of the probe. For a given pair of electrodes, the mutual information metric provides a numerical value of the mutual dependence of the signals of the pair. (e.g., if the signals are independent of each other, the metric is close to zero.)

The processor generates a graph with the electrodes as nodes, and with edges, as connections between the nodes, that exceed a selected mutual information metric threshold. In one embodiment the threshold is selected so that at least 50% of the electrodes have connections.

The processor calculates a local efficiency metric for each of the nodes, the local efficiency metric for a given node being a measure of how efficiently nodes connected to the given node exchange information. The processor then averages the local efficiency metric for the graph to generate a resultant local efficiency metric for the selected mutual information metric threshold.

The processor reiterates the steps of generating the graph, and averaging the respective local efficiency metrics while incrementing the selected mutual information metric threshold, so as to produce a set of ordered pairs of resultant local efficiency and mutual information threshold. The processor then analyzes the set of ordered pairs so as to classify the atrial fibrillation.

The analysis comprises estimating a percentage of remodeling of the heart tissue.

Detailed Description

In the following description, like elements in the drawings are identified by like numerals, and like elements are differentiated as necessary by appending a letter to the identifying numeral.

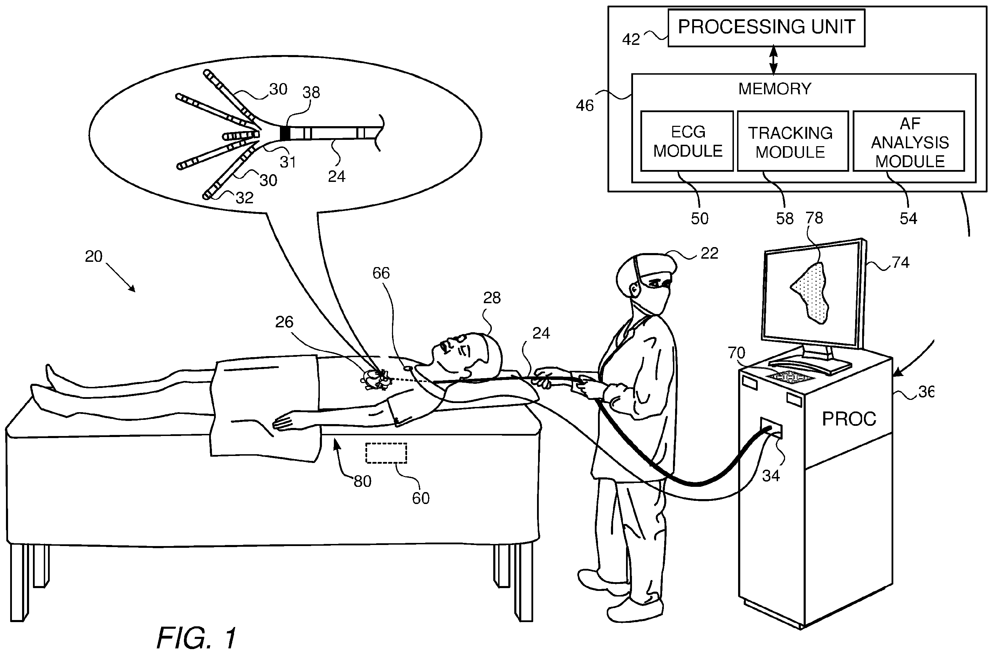

Reference is now made to Fig. 1, which is a schematic, pictorial illustration of an atrial fibrillation (AF) classification system 20. Fig. 1 depicts a physician 22 using a catheter 24, also referred to herein as a probe 24, to acquire unipolar or bipolar intra-cardiac ECG (electrocardiograph) signals from tissue of a heart 26 of a patient 28. Catheter 24 comprises, at its distal end 31, a plurality of spines 30, which may be mechanically flexible, and on each of the spines there are two or more electrodes 32. The electrodes 32 are located on spines 30 so that the separation between nearest-neighbor electrodes is less than 3 mm. While Fig. 1 depicts a catheter with five spines, other cases comprise catheters with other numbers of spines, as well as other catheters having a plurality of electrodes, where nearest-neighbor electrodes are separated by less than 3 mm.

Electrodes 32 are coupled, via conductors in catheter 24 and an interface 34, to a processor 36. Processor 36 comprises a processing unit 42, typically a central processing unit (CPU) and also referred to herein as CPU 42, which is coupled to a memory 46. Memory 46 comprises a number of modules: an ECG module 50, a tracking module 58, and an AF analysis module 54. The functions of the modules are described below.

CPU 42 typically comprises a general-purpose processor with software programmed to carry out the functions described herein. The software may be downloaded in electronic form, over a network, for example, or it may, alternatively or additionally, be provided and/or stored on non-transitory tangible media, such as magnetic, optical, or electronic memory.

Physician 22 communicates with processor 36 via an input device 70, such as a keypad or a pointing unit, as well as a screen 74, and the processor 36 may present results of procedures performed by the processor on the screen.

In system 20 CPU 42 may track the locations of electrodes 32 using tracking module 58. In one embodiment the CPU and the module are configured to implement an Advanced Current Location (ACL) system in system 20. The ACL system is described in US Patent 8,456,182. In the ACL system, a processor estimates the respective locations of the distal electrodes based on impedances or currents measured between each of distal electrodes 32 and a plurality of surface electrodes 66 that are coupled to the skin of patient 28. For ease of illustration, only one surface-electrode 66 is shown in Fig. 1. The processor may then associate any electrophysiological signal received from distal electrodes 32 with the location at which the signal was acquired.

Alternatively or additionally, the CPU and module 58 are configured to track the locations of electrodes 32 using an electromagnetic tracking system, such as is used in the Carto® System produced by Biosense Webster of Irvine California, and as is described in U.S. Patent Nos. 5,391,199 5,433,489 and 6,198,963. In this case, one or more magnetic sensors 38, typically single, double, or triple axis coils, are attached to distal end 31. In addition, a set 60 of alternating magnetic field radiators are located in proximity to, typically beneath, patient 28. Currents generated in sensors 38, in response to the fields from set 60, are registered by module 58, and the module and the CPU analyze the currents to determine the location of the distal end as well as the locations of electrodes 32, since the geometry of the distal end is known or may be estimated.

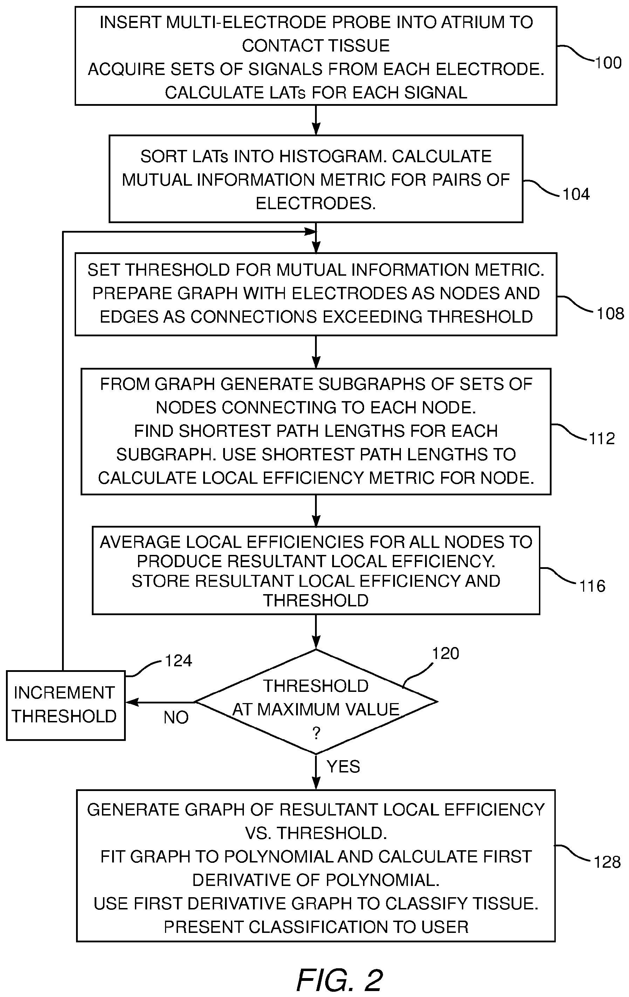

Fig. 2 is a flowchart of steps of an algorithm performed by processor 36, under overall control of physician 22. In an initial step 100 of the flowchart, physician 22 inserts probe 24 into patient 28 when the patient has atrial fibrillation. Processor 36 uses module 58 to track distal end 31 of the probe, and the physician observes the tracking of the probe on screen 74, and maneuvers the distal end until electrodes 32 of the probe contact a desired section of tissue within an atrium, herein assumed to comprise the left atrium, of heart 26.

Typically the tracking within the atrium is presented to the physician by processor 36 overlaying an icon of the probe on a pre-acquired map 78 of the atrium that is presented on screen 74. In addition to using the tracking provided by module 58, the physician may use processor 36 to measure impedances between electrodes 32 and a return electrode 80 attached to the skin of patient 28, and from the impedances confirm the contact of the electrodes with atrium tissue.

Once in position, physician 22 operates probe 24 to acquire and record sets of unipolar signals, i.e., sets of voltages measured with respect to electrode 80, for each electrode 32. Typically the sets of signals are acquired over a time period of approximately 2 minutes, i.e., for approximately 150 heartbeats, but signals may be acquired for shorter or longer periods. Typically, a minimum of 30 seconds of AF should be recorded. CPU 42 stores the acquired signals in memory 46, and for each signal the CPU uses ECG module 50 to calculate the local activations times (LATs). It will be understood that since patient 28 has atrial fibrillation, then, unlike a heart in sinus rhythm, the LAT values for one electrode, at a fixed position in the heart, have changing values, i.e., for the 150 heartbeats there may be 150 different LAT values.

In a first analysis step 104, CPU 42 uses AF analysis module 54 to sort the set of LAT values for each electrode into bins of a histogram. Typically, to simplify the calculations described hereinbelow, the histograms have equal-width bins. The histograms may be used to represent a distribution of the LAT values. In one example the number of bins is approximately $\sqrt{\frac{L}{5}}$ where L is the number of LAT values in the set.

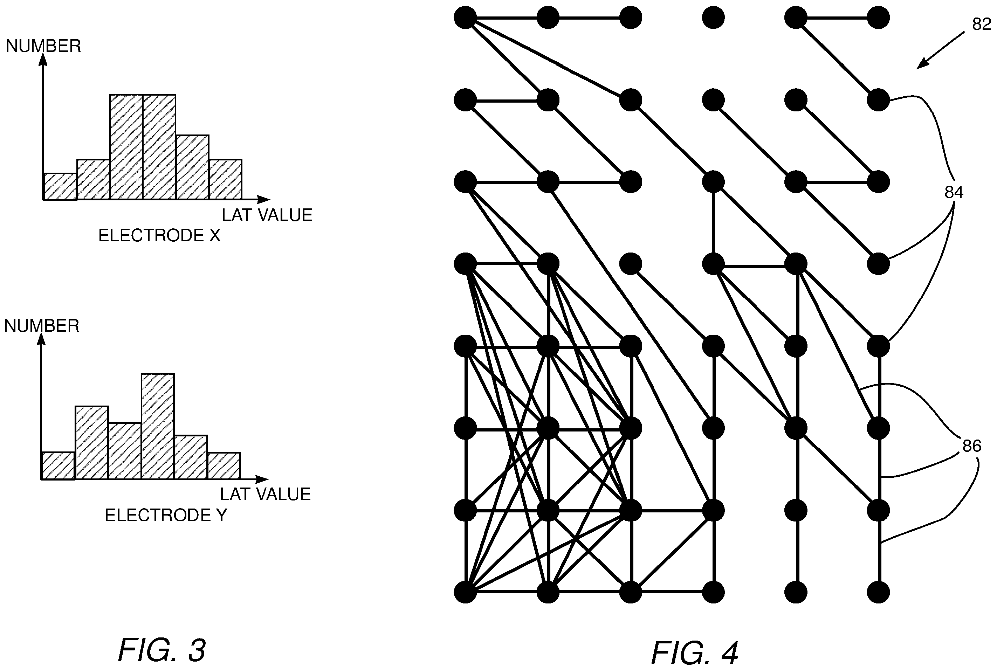

Fig. 3 illustrates two equal-width bin histograms, for electrodes 32X and 32Y, herein also referred to as electrodes X and Y. In the calculations performed by the CPU, the number of values in a given bin is assumed to give an estimate of the probability $\hat{p}$ of the occurrence of the mean LAT of the bin.

Returning to step 104 of the flowchart of Fig. 2, module 54 estimates a mutual information metric for multiple pairs of electrodes 32 according to equation (1):

$$I(X;Y) \approx \hat{I}(X;Y) = \frac{1}{N}\sum_{i=1}^{N}\log\frac{\hat{p}(x^i y^i)}{\hat{p}(x^i)\hat{p}(y^i)} \qquad (1)$$

where N represents the number of bins in the histograms,
X is a first given electrode,
Y is a second given electrode,

$\hat{p}(x^i) = \frac{x^i}{L}$ and $x^i$ is the number of LAT values in the $i^{th}$ bin of electrode X's histogram,

$\hat{p}(y^i) = \frac{y^i}{L}$ and $y^i$ is the number of LAT values in the $i^{th}$ bin of electrode Y's histogram,

L is the total number of values in each histogram, $\hat{p}(x^i y^i) = \frac{x^i y^i}{L^2}$

In the description hereinbelow, except where otherwise stated, the mutual information metric is estimated, using equation (1), for all pairs of electrodes 32. However, those having ordinary skill in the art will be able to adapt the description, *mutatis mutandis,* for embodiments where the metric is estimated for fewer than all pairs of electrodes 32, for example if some of the electrodes are not coupled to processor 36.

The mutual information metric is known in the art, and is a measure of dependence between two signals: i.e., the amount of information obtained about one signal based on the observation of another. Thus, if one signal is a deterministic function of another their mutual information is maximized; but if two signals are completely independent of each other, their mutual information is close to 0.

In a graph preparation step 108, CPU 42 uses module 54 to set a mutual information threshold such that a set percentage of electrode pairs have mutual information values above that value, and then prepares a graph, herein also termed a regional information graph, where nodes of the graph represent electrodes 32 and edges between the nodes represent connections that equal or exceed the threshold. Typically, the threshold is initially set to include 50% of electrode pairs.

Fig. 4 is an exemplary schematic regional information graph 82 prepared by CPU 42. By way of example in graph 82 there are 48 nodes 84, corresponding to the number of electrodes 32 in distal end 31 of the catheter. Edges 86 connect the nodes, corresponding to connections that are equal to or greater than a mutual information threshold.

In a subgraph step 112, for each given node in the regional information graph module 54 generates a subgraph, comprising a set of nodes that are connected to the given node being considered. CPU 42 calculates the shortest path between each pair of nodes in the set as the least number of connections between the pair, and for the calculation the CPU considers any given connection to have a unit length. The shortest path lengths are stored in memory 46.

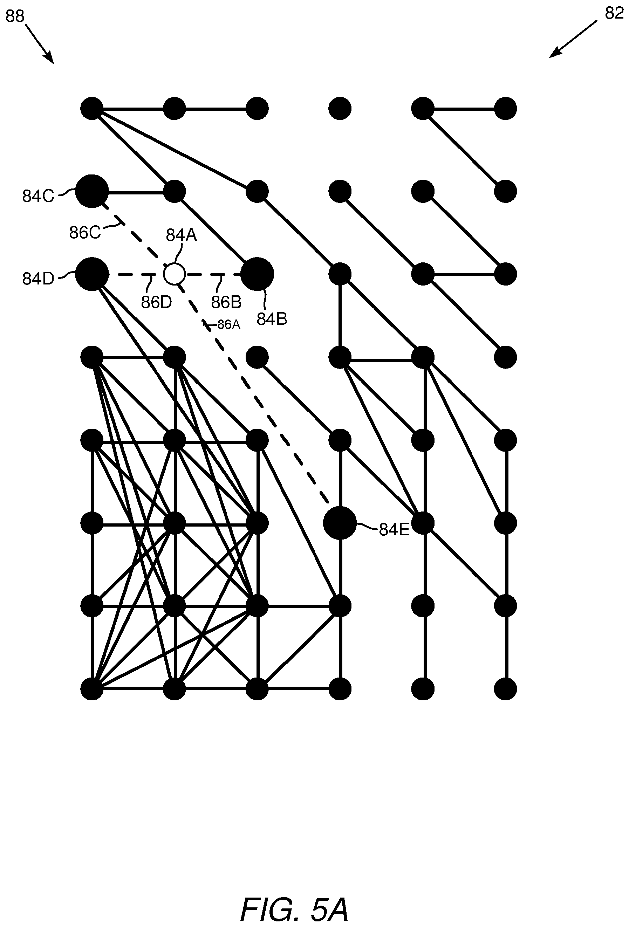

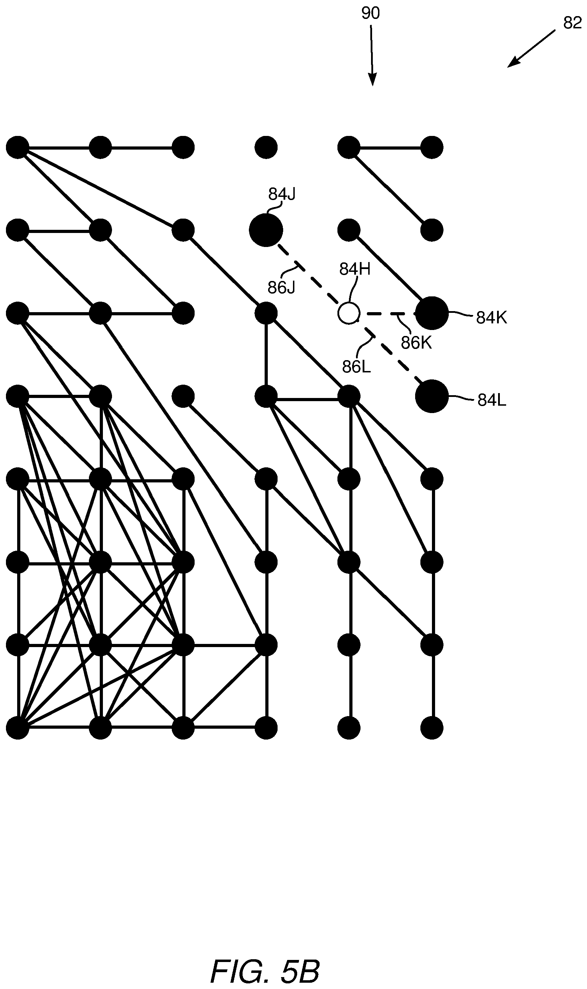

Figs. 5A and 5B are schematic diagrams of regional information graph 82, illustrating respectively a subgraph 88 for a node 84A, and a subgraph 90 for a node 84B.

As shown in Fig. 5A, node 84A has four connections or edges 86A, 86B, 86C, and 86D, respectively to nodes 84B, 84C, 84D, and 84E. Thus subgraph 88 for node 84A comprises the nodes 84B, 84C, 84D, and 84E. The shortest path lengths between pairs of these nodes may be determined from inspection of Fig. 5A, and is given in Table I:

Table I

| Node Pair | Shortest Path Length |
|-----------|---------------------|
| 84B-84C | 2 |
| 84B-84D | 12 |
| 84B-84E | 8 |
| 84C-84D | 12 |
| 84C-84E | 8 |
| 84D-84E | 4 |

As shown in Fig. 5B, node 84H has three connections or edges 86J, 86K, and 86L, respectively to nodes 84J, 84K, and 84L. Thus subgraph 90 for node 84H comprises the nodes 84J, 84K, and 84L. In contrast to node 84A, there are no paths between nodes of the subgraph of node 84H.

In step 112 the inverse of the shortest path lengths is used to calculate a local efficiency metric, $E_{local}$, for each node, according to equation (2):

$$E_{local} = \frac{1}{N_{Gi}(N_{Gi}-1)} \sum_{i \neq j \in Gi} \frac{1}{L_{i,j}} \qquad (2)$$

where $N_{Gi}$ is the number of nodes in a subgraph Gi, and
$L_{ij}$ is the shortest path length between nodes i and j in the subgraph Gi.

Local efficiency has been used in evaluating brains. It signifies fault tolerance, indicating how well each subgraph exchanges information when the central node is eliminated.

CPU 42 uses equation (2) to calculate a local efficiency metric for each node in the regional information graph. Thus, for node 84A equation (2), using the values for the path lengths of Table I, gives $E_{local}$ as 0.0972.

For node 84H, since there are no paths between nodes of the subgraph $E_{local}$ is 0.

In an averaging step 116 the calculated local efficiencies are averaged to provide a resultant local efficiency for the graph at the set mutual information threshold. CPU 42 stores the resultant local efficiency and the corresponding mutual information threshold as an ordered pair in memory 46.

As shown in a decision step 120 and an increment step 124, CPU 42 reiterates steps 108, 112, and 116, incrementing the mutual information metric threshold at each iteration, until a maximum value of the metric, typically approximately 95%, is reached. At each iteration the CPU stores the resultant local efficiency and the corresponding mutual information threshold as an ordered pair, so that when the iteration terminates, there is a set of ordered pairs available to CPU 42.

In a results step 128, from the set of ordered pairs produced in step 116, CPU 42 generates a graph of the resultant local efficiency vs. mutual information thresholds.

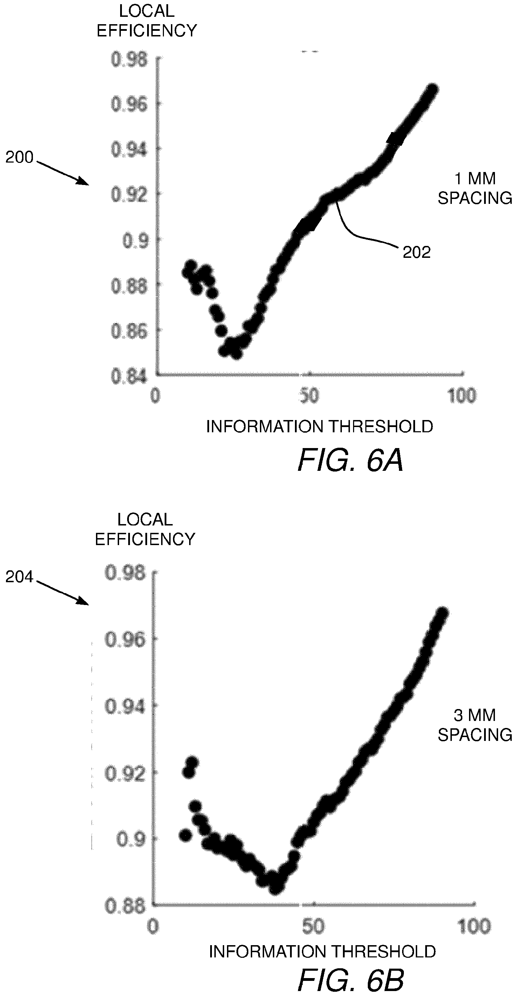

Figs. 6A and 6B are schematic graphs of resultant local efficiency vs. mutual information thresholds for catheters having different electrode spacings. In Fig. 6A a graph 200 illustrates simulated results for rotational signals acquired by a catheter with minimum spacing of electrodes 1 mm. In Fig. 6B a graph 204 illustrates simulated results for rotational signals acquired by a catheter with minimum spacing of electrodes 3 mm.

As is illustrated in graph 200, there is a plateau 202 where the slope of the graph decreases, compared to the slopes on either side of the plateau. There is no such plateau in graph 204. Graphs 200 and 204 illustrate that when electrodes are spaced by less than 3 mm, there is a plateau region in the graph when signals are rotational. This plateau is not present when the electrode spacing is 3 mm or greater.

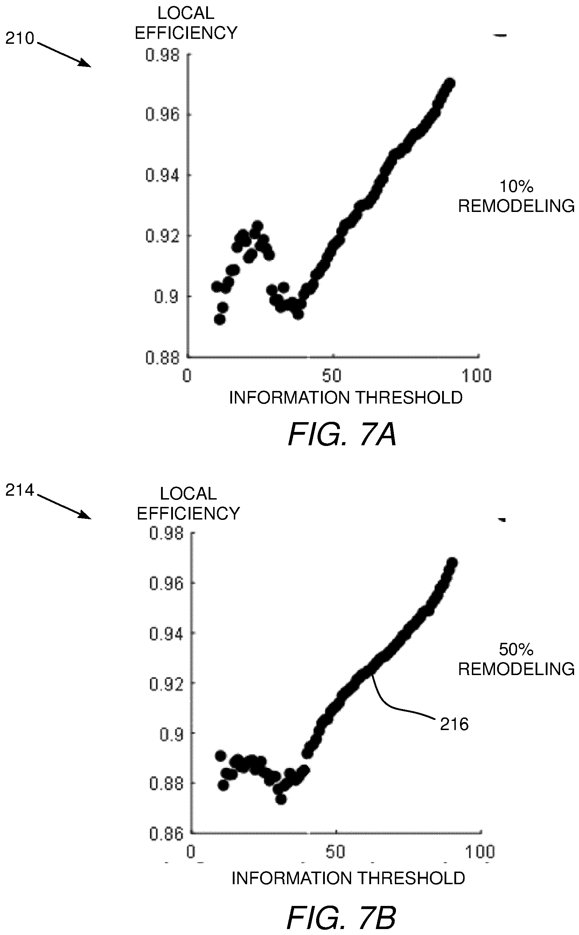

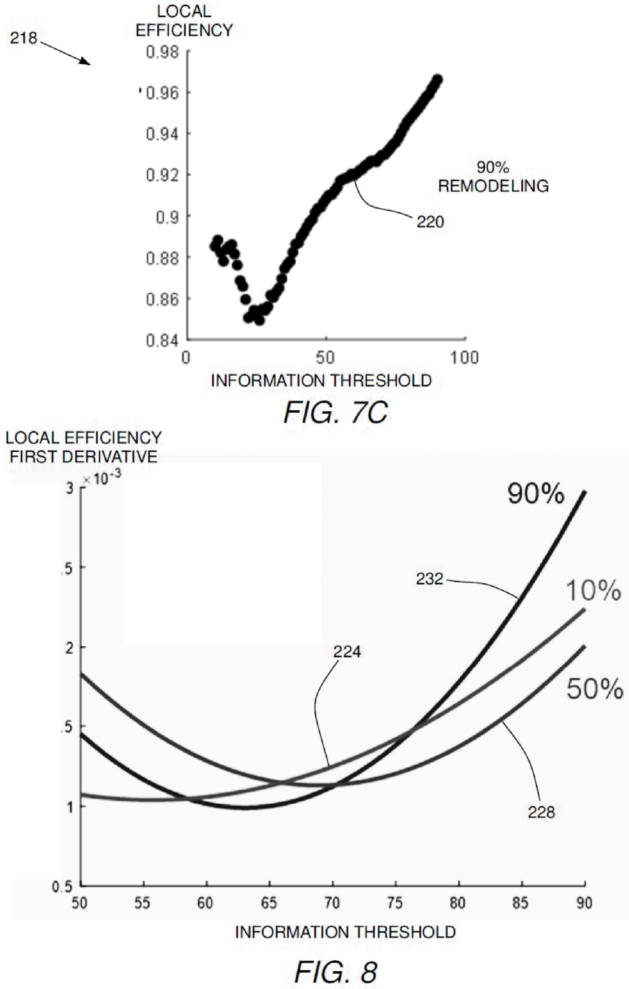

Figs. 7A, 7B, and 7C are schematic graphs of resultant local efficiency vs. mutual information thresholds for a catheter having minimum electrode spacings of 2 mm. The graphs are of simulated results for different percentages of remodeling of tissue of heart 26. A graph 210 is for 10% remodeling, a graph 214 is for 50% remodeling, and a graph 218 is for 90% remodeling. As is apparent from the graphs, 10% remodeling does not generate a plateau, whereas 50% remodeling and 90% remodeling respectively generate plateaus 216 and 220.

To illustrate the existence of a plateau more clearly, in step 128 CPU 42 fits a polynomial, typically a third degree polynomial to the generated graph, calculates the first derivative of the polynomial, and plots a graph of the first derivative vs. the information threshold.

Fig. 8 shows schematic graphs of local efficiency first derivatives vs. mutual information thresholds, generated from graphs 210, 214, and 218. To produce the graph of Fig. 8, CPU 42 fits a third degree polynomial to each of graphs 210, 214, and 218. The processing unit then calculates first derivatives of the local efficiency for each of the fitted polynomials, and plots the first derivatives vs. mutual information thresholds.

A graph 224 is the first derivative local efficiency vs. mutual information thresholds for the 10% remodeling graph 210;

A graph 228 is the first derivative local efficiency vs. mutual information thresholds for the 50% remodeling graph 214; and

A graph 232 is the first derivative local efficiency vs. mutual information thresholds for the 90% remodeling graph 218.

As seen in the graphs, the presence of a minimum in the graph indicates large or very large remodeling, whereas there is no minimum in the graph for small amounts, e.g., 10% remodeling. Furthermore, the "sharpness" of the minimum, e.g., the radius of curvature at the minimum, is indicative of the degree of remodeling, and the radius of curvature may be measured, or some other metric of sharpness, to evaluate the degree of modeling.

In step 128 the first derivative graph may be presented to physician 22 on screen 74. Alternatively or additionally, CPU 42 may determine whether or not there is a minimum in the first derivative graph, and if there is the CPU may measure its sharpness. From the determination the CPU may present a conclusion such as "no significant remodeling," "intermediate remodeling," or "high remodeling" on screen 74.

It will be appreciated that in step 128, there may be no requirement for CPU 42 to generate all the physical graphs described, and that the CPU may generate data corresponding to some of the graphs. In other words, the CPU may just use the ordered pairs from step 116, and from the ordered pairs may present the first derivative graph and/or the conclusion described above.

The description above has, for simplicity, assumed that the signals acquired by the electrodes are used to form LATs. Those having ordinary skill in the art will be able to adapt the description, *mutatis mutandis,* using voltages, typically unipolar or bipolar voltages, or action potential voltages, of the signals, rather than LATs.

It will be appreciated that the embodiments described above are cited by way of example, and that the present invention is not limited to what has been particularly shown and described hereinabove. The scope of the present invention is defined by the appended claims and includes both combinations and subcombinations of the various features described hereinabove, as well as variations and modifications thereof, which fall within the scope of the claims.

**Claims**

1.  A computer program comprising instructions that, when executed by a processor in communication with a plurality of electrodes in contact with heart tissue undergoing atrial fibrillation, configure the processor to:

    acquire, from the plurality of electrodes, respective signals;
    calculate, from the signals, respective mutual information metrics between multiple pairs of the electrodes;
    generate a graph with the electrodes as nodes, and edges as connections therebetween for which the respective mutual information metrics exceed a selected mutual information metric threshold;

calculate a respective local efficiency metric for each node, indicating an efficiency of information exchange between the node and other nodes connected to the node, based on path lengths between the connected nodes; and

average respective local efficiency metrics of the nodes to formulate a resultant local efficiency for the selected mutual information metric threshold; **characterized in that** the instructions further configure the processor to: analyze the resultant local efficiency and the selected mutual information metric threshold to classify the atrial fibrillation based on estimating a percentage of remodeling of the heart tissue.

2. The computer program according to claim 1, wherein the instructions further configure the processor to present to a user of the processor a classification of the atrial fibrillation.

3. Apparatus, comprising:

a probe, having a plurality of electrodes configured to contact heart tissue undergoing atrial fibrillation; and a processor, configured to:

receive signals from the electrodes and calculate, from the signals, mutual information metrics between multiple pairs of the electrodes;

generate a graph with the electrodes as nodes, and edges as connections therebetween for which the respective mutual information metrics exceed a selected mutual information metric threshold;

calculate a respective local efficiency metric for each node, indicating an efficiency of information exchange between the node and other nodes connected to the node, based on path lengths between the connected nodes; and

average respective local efficiency metrics of the nodes to formulate a resultant local efficiency for the selected mutual information metric threshold; **characterized in that** the processor is further configured to:

analyze the resultant local efficiency and the selected mutual information metric threshold to classify the atrial fibrillation based on estimating a percentage of remodeling of the heart tissue.

4. The computer program of claim 1 or the apparatus according to claim 3, wherein the signals are unipolar or bipolar voltage or action potential voltage vs. time signals.

5. The computer program of claim 1 or the apparatus according to claim 3, wherein the step of calculating from the signals respective mutual information metrics between multiple pairs of the electrodes comprises estimating local activation times (LATs) from the signals.

6. The computer program of claim 1 or the apparatus according to claim 3, wherein the heart tissue is part of an atrium, and wherein classifying the atrial fibrillation comprises estimating a percentage of remodeling of the atrium.

7. The apparatus according to claim 3, wherein the processor is configured to present to a user of the apparatus a classification of the atrial fibrillation.

8. The computer program of claim 1 or the apparatus according to claim 3, wherein the plurality of electrodes are located on a catheter having a multiplicity of spines.

9. The computer program of claim 1 or the apparatus according to claim 3, wherein nearest-neighbor electrodes comprised in the plurality of electrodes are separated by less than 3mm.

10. The computer program of claim 1 or the apparatus according to claim 3, wherein averaging respective local efficiency metrics of the nodes comprises generating subgraphs of nodes connected directly to a given node, calculating local efficiency metrics for each of the subgraphs, and averaging the calculated local efficiency metrics.

11. The computer program of claim 1 or the apparatus according to claim 3, wherein the processor is further configured to reiterate the steps of generating the graph, and averaging the respective local efficiency metrics while incrementing the selected mutual information metric threshold, so as to produce a set of ordered pairs of resultant local efficiency and mutual information threshold.

**12.** The computer program or apparatus according to claim 11, wherein the processor is further configured to analyze the set of ordered pairs to classify the atrial fibrillation.

**13.** The computer program or apparatus according to claim 12, wherein analyzing the set of ordered pairs comprises fitting a polynomial to the set, and classifying the atrial fibrillation in response to calculating a first derivative of the polynomial.

**14.** The computer program of claim 1 or the apparatus according to claim 3, wherein calculating from the signals comprises calculating, from the signals, respective mutual information metrics between all pairs of the electrodes.

**Patentansprüche**

**1.** Computerprogramm, umfassend Anweisungen, die, wenn sie durch einen Prozessor in Kommunikation mit einer Vielzahl von Elektroden in Berührung mit einem Herzgewebe, das ein Vorhofflimmern erfährt, ausgeführt werden, den Prozessor konfigurieren zum:

Erfassen, von der Vielzahl von Elektroden, jeweiliger Signale;
Berechnen, aus den Signalen, jeweiliger Transinformationsmetriken zwischen mehreren Paaren der Elektroden;
Erzeugen eines Graphen mit den Elektroden als Knoten und Kanten als Verbindungen dazwischen, für die die jeweiligen Transinformationsmetriken eine ausgewählte Transinformationsmetrikschwelle überschreiten;
Berechnen einer jeweiligen Metrik einer lokalen Effizienz für jeden Knoten, die eine Effizienz eines Informationsaustauschs zwischen dem Knoten und anderen mit dem Knoten verbundenen Knoten angibt, basierend auf Pfadlängen zwischen den verbundenen Knoten; und
Mitteln der jeweiligen Metriken der lokalen Effizienz der Knoten, um eine resultierende lokale Effizienz für die ausgewählte Transinformationsmetrikschwelle zu formulieren; **dadurch gekennzeichnet, dass** die Anweisungen den Prozessor ferner konfigurieren zum:
Analysieren der resultierenden lokalen Effizienz und der ausgewählten Transinformationsmetrikschwelle, um das Vorhofflimmern basierend auf einem Schätzen eines Prozentsatzes eines Remodelings des Herzgewebes zu klassifizieren.

**2.** Computerprogramm nach Anspruch 1, wobei die Anweisungen den Prozessor ferner konfigurieren, um einem Benutzer des Prozessors eine Klassifizierung des Vorhofflimmerns zu präsentieren.

**3.** Einrichtung, umfassend:

eine Sonde, die eine Vielzahl von Elektroden aufweist, die konfiguriert sind, um das Herzgewebe, das das Vorhofflimmern erfährt, zu berühren; und
einen Prozessor, der konfiguriert ist zum:

Empfangen von Signalen von den Elektroden und Berechnen, aus den Signalen, von Transinformationsmetriken zwischen mehreren Paaren der Elektroden;
Erzeugen eines Graphen mit den Elektroden als Knoten und Kanten als Verbindungen dazwischen, für die die jeweiligen Transinformationsmetriken eine ausgewählte Transinformationsmetrikschwelle überschreiten;
Berechnen einer jeweiligen Metrik der lokalen Effizienz für jeden Knoten, die eine Effizienz des Informationsaustauschs zwischen dem Knoten und anderen mit dem Knoten verbundenen Knoten angibt, basierend auf Pfadlängen zwischen den verbundenen Knoten; und
Mitteln der jeweiligen Metriken der lokalen Effizienz der Knoten, um eine resultierende lokale Effizienz für die ausgewählte Transinformationsmetrikschwelle zu formulieren; **dadurch gekennzeichnet, dass** der Prozessor ferner konfiguriert ist zum:

Analysieren der resultierenden lokalen Effizienz und der ausgewählten Transinformationsmetrikschwelle, um das Vorhofflimmern basierend auf dem Schätzen eines Prozentsatzes des Remodelings des Herzgewebes zu klassifizieren.

**4.** Computerprogramm nach Anspruch 1 oder Einrichtung nach Anspruch 3, wobei die Signale Signale einer unipolaren oder bipolaren Spannung oder einer Aktionspotentialspannung gegen Zeit sind.

**5.** Computerprogramm nach Anspruch 1 oder Einrichtung nach Anspruch 3, wobei der Schritt des Berechnens, aus den Signalen, jeweiliger Transinformationsmetriken zwischen mehreren Paaren der Elektroden das Schätzen lokaler Aktivierungszeiten (LATs) aus den Signalen umfasst.

**6.** Computerprogramm nach Anspruch 1 oder Einrichtung nach Anspruch 3, wobei das Herzgewebe Teil eines Vorhofs ist und wobei ein Klassifizieren des Vorhofflimmerns das Schätzen eines Prozentsatzes des Remodelings des Vorhofs umfasst.

**7.** Einrichtung nach Anspruch 3, wobei der Prozessor konfiguriert ist, um einem Benutzer der Einrichtung eine Klassifizierung des Vorhofflimmerns zu präsentieren.

**8.** Computerprogramm nach Anspruch 1 oder Einrichtung nach Anspruch 3, wobei die Vielzahl von Elektroden auf einem Katheter, der eine Vielzahl von Dornen aufweist, angeordnet sind.

**9.** Computerprogramm nach Anspruch 1 oder Einrichtung nach Anspruch 3, wobei nächstbenachbarte Elektroden, die in der Vielzahl von Elektroden enthalten sind, um weniger als 3 mm voneinander getrennt sind.

**10.** Computerprogramm nach Anspruch 1 oder Einrichtung nach Anspruch 3, wobei das Mitteln der jeweiligen Metriken der lokalen Effizienz der Knoten das Erzeugen von Untergraphen von Knoten, die direkt mit einem gegebenen Knoten verbunden sind, das Berechnen der Metriken der lokalen Effizienz für jeden der Untergraphen und das Mitteln der berechneten Metriken der lokalen Effizienz umfasst.

**11.** Computerprogramm nach Anspruch 1 oder Einrichtung nach Anspruch 3, wobei der Prozessor ferner konfiguriert ist, um die Schritte des Erzeugens des Graphen und des Mittelns der jeweiligen Metriken der lokalen Effizienz zu wiederholen, während die ausgewählte Transinformationsmetrikschwelle erhöht wird, um einen Satz geordneter Paare aus resultierender lokaler Effizienz und Transinformationsschwelle zu produzieren.

**12.** Computerprogramm oder Einrichtung nach Anspruch 11, wobei der Prozessor ferner konfiguriert ist, um den Satz geordneter Paare zu analysieren, um das Vorhofflimmern zu klassifizieren.

**13.** Computerprogramm oder Einrichtung nach Anspruch 12, wobei das Analysieren des Satzes geordneter Paare ein Anpassen eines Polynoms an den Satz und das Klassifizieren des Vorhofflimmerns als Reaktion auf das Berechnen eines ersten Derivats des Polynoms umfasst.

**14.** Computerprogramm nach Anspruch 1 oder Einrichtung nach Anspruch 3, wobei das Berechnen, aus den Signalen, das Berechnen, aus den Signalen, jeweiliger Transinformationsmetriken zwischen allen Paaren der Elektroden umfasst.

**Revendications**

**1.** Programme informatique comprenant des instructions qui, lorsqu'elles sont exécutées par un processeur en communication avec une pluralité d'électrodes en contact avec le tissu cardiaque subissant une fibrillation auriculaire, configurent le processeur pour :

acquérir, à partir de la pluralité d'électrodes, des signaux respectifs ;
calculer, à partir des signaux, des métriques d'information mutuelle respectives entre de multiples paires d'électrodes ;
générer un graphe avec les électrodes en tant que nœuds et des arêtes en tant que connexions entre ceux-ci pour lesquelles les métriques d'information mutuelle respectives dépassent un seuil de métrique d'information mutuelle sélectionné ;
calculer une métrique d'efficacité locale respective pour chaque nœud, indiquant une efficacité d'échange d'informations entre le nœud et d'autres nœuds connectés au nœud, sur la base de longueurs de chemin entre les nœuds connectés ; et
calculer une moyenne des métriques d'efficacité locale respectives des nœuds afin de formuler une efficacité locale résultante pour le seuil de métrique d'information mutuelle sélectionné ; **caractérisé en ce que** les instructions configurent en outre le processeur pour :
analyser l'efficacité locale résultante et le seuil de métrique d'information mutuelle sélectionné pour classifier la fibrillation auriculaire sur la base de l'estimation d'un pourcentage de remodelage du tissu cardiaque.

2. Programme informatique selon la revendication 1, dans lequel les instructions configurent en outre le processeur pour présenter à un utilisateur du processeur une classification de la fibrillation auriculaire.

3. Appareil, comprenant :

une sonde ayant une pluralité d'électrodes configurées pour entrer en contact avec un tissu cardiaque subissant une fibrillation auriculaire ; et
un processeur, configuré pour :

recevoir des signaux depuis les électrodes et calculer, à partir des signaux, des métriques d'information mutuelle entre de multiples paires d'électrodes ;
générer un graphique avec les électrodes en tant que nœuds et des arêtes en tant que connexions entre ceux-ci pour lesquelles les métriques d'information mutuelle respectives dépassent un seuil de métrique d'information mutuelle sélectionné ;
calculer une métrique d'efficacité locale respective pour chaque nœud, indiquant une efficacité d'échange d'informations entre le nœud et d'autres nœuds connectés au nœud, sur la base de longueurs de chemin entre les nœuds connectés ; et
établir une moyenne des métriques d'efficacité locale respectives des nœuds afin de formuler une efficacité locale résultante pour le seuil de métrique d'information mutuelle sélectionné ; **caractérisé en ce que** le processeur est en outre configuré pour :
analyser l'efficacité locale résultante et le seuil de métrique d'information mutuelle sélectionné pour classifier la fibrillation auriculaire sur la base de l'estimation d'un pourcentage de remodelage du tissu cardiaque.

4. Programme informatique selon la revendication 1 ou appareil selon la revendication 3, dans lequel les signaux sont des signaux de tension ou de tension de potentiel d'action en fonction du temps, unipolaires ou bipolaires.

5. Programme informatique selon la revendication 1 ou appareil selon la revendication 3, dans lequel l'étape consistant à calculer, à partir des signaux, des métriques d'information mutuelle respectives entre de multiples paires d'électrodes comprend l'estimation de temps d'activation locaux (LAT) à partir des signaux.

6. Programme informatique selon la revendication 1 ou appareil selon la revendication 3, dans lequel le tissu cardiaque fait partie d'une oreillette, et dans lequel la classification de la fibrillation auriculaire comprend l'estimation d'un pourcentage de remodelage de l'oreillette.

7. Appareil selon la revendication 3, dans lequel le processeur est configuré pour présenter à un utilisateur de l'appareil une classification de la fibrillation auriculaire.

8. Programme informatique selon la revendication 1 ou appareil selon la revendication 3, dans lequel la pluralité d'électrodes sont situées sur un cathéter ayant une multiplicité de branches.

9. Programme informatique selon la revendication 1 ou appareil selon la revendication 3, dans lequel les électrodes voisines les plus proches comprises dans la pluralité d'électrodes sont séparées de moins de 3 mm.

10. Programme informatique selon la revendication 1 ou appareil selon la revendication 3, dans lequel la moyenne des métriques d'efficacité locale respectives des nœuds comprend la génération de sous-graphes de nœuds connectés directement à un nœud donné, le calcul de métriques d'efficacité locale pour chacun des sous-graphes et le calcul de la moyenne des métriques d'efficacité locale calculées.

11. Programme informatique selon la revendication 1 ou appareil selon la revendication 3, dans lequel le processeur est en outre configuré pour réitérer les étapes de génération du graphe et de calcul de la moyenne des métriques d'efficacité locale respectives tout en augmentant le seuil de métrique d'information mutuelle sélectionné, de manière à produire un ensemble de paires ordonnées d'efficacité locale résultante et de seuil d'information mutuelle.

12. Programme informatique ou appareil selon la revendication 11, dans lequel le processeur est en outre configuré pour analyser l'ensemble de paires ordonnées afin de classifier la fibrillation auriculaire.

**13.** Programme informatique ou appareil selon la revendication 12, dans lequel l'analyse de l'ensemble de paires ordonnées comprend l'ajustement d'un polynôme à l'ensemble, et la classification de la fibrillation auriculaire en réponse au calcul d'une première dérivée du polynôme.

**14.** Programme informatique selon la revendication 1 ou appareil selon la revendication 3, dans lequel le calcul à partir des signaux comprend le calcul, à partir des signaux, de métriques d'information mutuelle entre toutes les paires des électrodes.

FIG. 1

EP 4 029 448 B1

FIG. 2

FIG. 3

FIG. 4

EP 4 029 448 B1

FIG. 5A

*FIG. 5B*

FIG. 6A

FIG. 6B

FIG. 7A

FIG. 7B

FIG. 7C

FIG. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 8456182 B
- US 5391199 A
- US 5433489 A
- US 6198963 B

### Non-patent literature cited in the description

- **TAO S et al.** Ablation as targeted perturbation to rewire communication network of persistent atrial fibrillation. *PLoS ONE*, 05 July 2017, vol. 12 (7), e0179459